# EUROPEAN PATENT SPECIFICATION

(11) **EP 2 735 288 B1**
(45) Date of publication and mention of the grant of the patent: **11.01.2017**
(21) Application number: 12194348.4
(22) Date of filing: 27.11.2012
(51) Int. Cl.: A61F 5/00

(54) **Obesity reduction aide**
Adipositasreduktionshilfe
Aide de réduction de l'obésité

(43) Date of publication of application: 28.05.2014
(73) Proprietor: Schicker, Matthias, 65719 Hofheim a. Ts. (DE)
(72) Inventor: Schicker, Matthias, 65719 Hofheim a. Ts. (DE)
(74) Representative: Ricker, Mathias

(56) References cited:
- DE-A1-102009 009 916
- US-A- 5 899 691
- US-A1- 2003 059 737
- US-A1- 2007 048 347

## Description

### Field of the invention

The invention relates to devices used to reduce the obesity of a subject, in particular the invention relates to devices for modifying the chewing of a subject.

### Background and related art

There may be a variety of reasons why a person may be obese. Sometimes obesity is caused by a medical condition. Often obesity is caused by a combination of poor eating habits and/or lifestyle choices.

United States patent application publication US 2003/0059737 A1 discloses a dieting device. The device comprises at least one pair of frames which are secured to the maxillary and mandibular dentitions. One of the frames has mounted to the side thereof one or more permanent split pole magnets which magnetically attract either a corresponding set of magnets of a ferromagnetic mass mounted to one side of the corresponding frame. The magnetic attraction between upper and lower dentitions to which the frames have been applied is not so great as to be impossible to overcome by the wearer and thus the jaws can be opened in an emergency, but in use the frames will make mastication, and thus oral ingestion of food, difficult.

### Summary

The invention provides for: a device herein referred to as an obesity reduction aid, an obesity reduction system, and a method of modifying an obesity reduction aid in the independent claims. Embodiments are given in the dependent claims.

Many people try to treat obesity by dieting. Dieting is, however, often unsuccessful. After initial success, the dieter's weight will go through the well known yo-yo effect and the dieter may eventually wind up even more obese than before. The dieting device described in US 2003/0059737 A1 makes it more difficult to eat, thus aiding the dieting process.

The invention may provide for a more permanent means of helping obese people to lose weight. An upper and a lower plate are operable to be attached to the teeth of a subject. The upper plate has a reduced surface area with respect to the lower plate. The effect of this is that the subject finds it more difficult to chew his or her food, however the motion of the jaw is not restricted as in US 2003/0059737 A1. Embodiments of the invention may have the effect training a subject to chew longer and more thoroughly. This may have a significant effect on obese people.

When one eats there is a delay in the time between when we have eaten and when we feel full. Normally people eat slowly enough that when they have eaten enough food, they stop eating. Obese people may have the problem that they gobble their food. They eat the food too rapidly with the effect that by the time they feel full, they have already overeaten. When a subject wears an obesity reduction aid according to an embodiment of the invention, they may be forced to chew more and eat more slowly. This additional delay in ingesting the food may prevent them from overeating. Additionally experiments have shown that subjects who wear an obesity reduction aid when eating for 10 to 12 weeks have demonstrated better chewing technique and experienced weight loss even after they stop wearing the obesity reduction aid. They were trained to chew their food longer before swallowing. They did not need to diet, because the added delay in eating made them feel full and they stopped eating naturally.

In one aspect the invention provides for an obesity reduction aid that comprises a lower plate adapted for covering the mandibular teeth of a subject. The lower plate comprises a first elastic material. In some embodiments the lower plate is made entirely of the first elastic material. The lower plate has a first chewing surface. The first chewing surface is smooth. The first chewing surface follows an average of the occlusal surface of the mandibular teeth of the subject. An occlusal surface of the teeth is a surface of the teeth that is used to chew. An average of the occlusal surface would be a smooth surface which approximates the rough occlusal surface of the teeth. That is to say that the first chewing surface follows an average of the occlusal surface of the mandibular teeth and by this it may be interpreted that the chewing surface forms a surface which is offset from the occlusal plane. The occlusal plane may also be referred to as the bite plane.

The lower plate is essentially a mouthpiece made of an elastic material which covers the mandibular teeth. It provides a first chewing surface which may be offset from an average of the occlusal surface of the subject. In some embodiments the first chewing surface may follow or be offset from the occlusal plane. The obesity reduction aid further comprises an upper plate adapted for covering the maxillary teeth of the subject. The upper plate comprises a second elastic material. In some embodiments the upper plate may be made entirely of the second elastic material. The upper plate has a second chewing surface.

The first chewing surface mates with the second chewing surface. By mating it means that the second chewing surface has a surface which interfaces or matches the first chewing surface such that the upper plate and the lower plate can be put together. The second chewing surface is formed by a protruding ridge. The surface area of the first chewing surface is larger than the surface area of the second chewing surface. The surface area of the second chewing surface is smaller. This has the effect that when the obesity reduction aid is worn by the subject the chewing ability of the subject is reduced. This may have the benefit that the subject needs to chew his or her food longer before it is completely masticated. The effect of this is that the subject may learn to chew his or her food more slowly. This causes food ingested by the subject to be eaten more slowly.

A common cause of extreme obesity is when the subject gobbles his or her food. That is the subject eats food so quickly that the body's normal regulation of how much food to eat is circumvented. For instance typically when one begins to eat there is approximately a 20 minute delay between eating and the effect of feeling full. If someone gobbles food they may ingest a large amount of food, perhaps much more than is necessary before the subject begins to feel full. The obesity reduction aid may have the benefit that it slows down the eating process and prevents the subject from gobbling the food. As the food is in the subject's mouth longer the subject may also have the benefit of enjoying the flavor of the food more. When worn over an extended period of time, say perhaps for a month or several months, the subject learns to chew his or her food more thoroughly and over a longer period of time.

After a period of time it may no longer be necessary for the subject to wear the obesity reduction aid during eating. The obesity reduction aid may therefore be used in training the subject to chew more and eat more slowly. This may result in a significant weight loss by a subject without dieting. The body's natural mechanism for feeling full will take over and the subject will eat a normal amount of food and therefore lose weight.

In an embodiment the width of the first chewing surface is wider than the width of the second chewing surface. Essentially the surface area of the upper teeth have a surface area used for chewing which is reduced.

The first and second elastic materials may be identical. The first and second elastic materials may also be different materials.

In another embodiment the first chewing surface may be defined by the curve of Spee. The curve of Spee is a curve which defines a surface upon which the mandible exerts an even force by the teeth. For instance if one were to stick an object into the mouth the teeth would contact this object and all of the force would be directed towards this object. However, if the object with a surface along the curve of Spee is inserted into the mouth the force would be evenly distributed along this curve.

Embodiments of the invention may further have the benefit that they can be worn with crowns, bridges and other dental prosthetic devices. The elastic material may simply fit around these prosthetic structures and enable the subject to simply insert the lower and upper plates and eat.

The protruding ridge may have a step-like cross-section.

In another embodiment the protruding ridge is continuous along a path following the maxillary teeth of the subject. The upper plate may be a mouthpiece that is inserted into the mouth. The mouthpiece may have a U-shaped structure. The protruding ridge may follow along the entire length of this U-shaped structure.

In another embodiment the protruding ridge is continuous along a path following the incisors of the maxillary teeth of the subject. This embodiment may be beneficial because if the protruding ridge had gaps where the incisors of the subject are, then it may cause the subject to speak with a lisp.

It should be noted that in some of the claims the position of the ridge or other structures is made with relation to the teeth of the subject. This is appropriate as the lower plate and the upper plate are each mouthpieces which are molded to fit or cover the teeth of the subject. There may be an indentation or space for receiving the teeth in each of the lower plate and the upper plate. The structure of the subject's teeth would therefore be imprinted into the lower plate and the upper plate. The other structures of the obesity reduction aid can then be paired to the location of the various teeth of the subject.

In another embodiment the second chewing surface has an average width. The protruding ridge has a width between one fifth to four fifths of the average width.

In another embodiment the protruding ridge has a width between one third to two thirds of the average width.

In another embodiment the protruding ridge has a width between one quarter to three quarters of the average width.

In another embodiment the protruding ridge is at least partially discontinuous in the molar region of the subject. For instance a portion of the protruding ridge may be removed in the molar region. This may have the effect of making it more difficult to chew. For instance if the subject is not chewing slowly enough and/or is not losing weight quickly enough material from the protruding ridge in the molar region can be removed.

In another embodiment at least a portion of the second chewing surface has a convex profile. At least a portion of the first chewing surface has a concave profile. This may have the benefit that the concave and convex profiles fit together and serve to help align the subject's teeth properly. This may reduce the likelihood that there are side forces which tend to put side forces on the teeth. It may also be an aid for helping the subject chew properly by helping to align the lower plate and the upper plate.

In another embodiment the first chewing surface has a concave profile along its entire length. In this embodiment either the entire second chewing surface has a convex profile or only a portion of it. For instance if part of the protruding ridge in the molar region had been removed then of course the material which has been removed would not have a convex profile.

In another embodiment the first chewing surface is above the occlusal plane. The first chewing surface spans the occlusal surface. The teeth in the molar region have cusps. The high points of the outer cusps may define where the occlusal surface is. The occlusal plane is a plane or surface which goes between the cusps and is concave. The first chewing surface does not cross the occlusal surface in this embodiment.

In another embodiment the first chewing surface has a first curvature. The second chewing surface has a second curvature. The first curvature is equal to the second curvature. This may be interpreted as the first chewing surface mating with the second chewing surface. In some embodiments the first and second curvatures may not be constant along the entire length of the lower plate and the upper plate. However, it is understood that when the lower plate and the upper plate are installed into a subject the curvatures at various points along the upper and lower plates will match each other.

In another embodiment the curvature is defined by the cusps of the mandibular molars of the subject. Essentially the concave profile may be defined by the cusps of the teeth.

In another embodiment the first chewing surface has a concave profile. The lower plate has a first tooth mounting surface. The first tooth mounting surface approximates the surface of the cusps of the mandibular teeth of the subject. The first tooth mounting surface may also be interpreted as fitting around the mandibular teeth of the subject. The concave profile is defined by the cusps of the teeth. The lower plate is formed from a single molded piece of the first elastic material. The upper plate is formed from a single molded piece of the second elastic material. The lower plate comprises at least one first retention clip made from the first elastic material. The upper plate comprises a second retention clip made from the second elastic material. The first elastic material has an elastic modulus of between 2000 and 4000 MPa. The elastic module is preferably between 2600 and 3000 MPa. The second elastic material has an elastic modulus of between 2000 and 4000 MPa. The second elastic material preferably has an elastic modulus of between 2600 and 3000 MPa. The first chewing surface is defined by the curve of Spee.

In another embodiment the lower plate comprises at least one first retention clip made from the first elastic material. A retention clip as used herein encompasses a structure operable for holding a upper or lower plate to a tooth or teeth.

In another embodiment the upper plate comprises a second retention clip made from the second elastic material.

In another embodiment the lower plate is formed from a single molded piece of the first elastic material.

In another embodiment the upper plate is formed from a single molded piece of the second elastic material.

In another embodiment the upper and/or lower plate are colored to match a tooth color of the subject.

In another embodiment the first elastic material has an elastic modulus of between 2000 and 4000 MPa and/or the second elastic material has an elastic modulus of between 2000 and 4000 MPa. Each of the first and second elastic materials has preferably an elastic modulus between 2600 and 3000 MPa. A thermoplastic such as a polyacetal-copolymer with an elastic modulus of approximately 2800 MPa may work well.

In another embodiment the obesity reduction aid comprises an RFID chip. The RFID chip is operable for storing any one of the following: a patient identifier, a patient identity, a current weight, a starting weight, an ending weight, a weight history database, a three-dimensional obesity reduction aid model, and combinations thereof. The RFID chip may be molded into the upper or lower plate. In some embodiments an area of the upper or lower plate is removed, the RFID chip is installed and then a third elastic material is used to seal the RFID chip into either the upper plate or the lower plate. The third elastic material may in some instances be identical with the first or second elastic material.

In another aspect the invention provides for an obesity reduction system comprising an obesity reduction aid according to any one of the preceding claims. The obesity reduction system further comprises a computer memory for storing computer-executable instructions and for storing a weight database. The weight database comprises measurements of the subject's weight over a period of time. The computer memory may be within a computer, or a portion of the memory such as for storing the weight database may be located in a different location. For instance the weight database may be stored in an RFID chip which is installed into the obesity reduction aid. The obesity reduction system further comprises a processor for executing the instructions.

Execution of the instructions causes the process to record a subject's weight in the weight database. Execution of the instructions further causes the processor to determine a chewing surface area reduction recommendation. This is performed using a model of the first chewing surface, the second chewing surface and the weight database. Execution of the instructions further causes the processor to display the surface area reduction recommendation on a display. This for instance may be useful for a dentist or other healthcare provider to reduce the surface area of the protruding ridge to make it more difficult for the subject to chew. For instance if the subject is not losing weight or is not losing weight quickly enough the healthcare provider may use the obesity reduction system to determine how much to reduce the surface area of the protruding ridge. The chewing surface area reduction recommendation may for instance be formed using rules which are predetermined or may be determined using a model which is determined empirically.

In another embodiment the obesity reduction system further comprises a scale. The subject weight is read automatically by the processor using the scale.

A 'computer-readable storage medium' as used herein encompasses any tangible storage medium which may store instructions which are executable by a processor of a computing device and/or store data accessible to the processor. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, punched tape, punch cards, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. References to a computer-readable storage medium should be interpreted as possibly being multiple computer-readable storage mediums. Various executable components of a program or programs may be stored in different locations. The computer-readable storage medium may for instance be multiple computer-readable storage medium within the same computer system. The computer-readable storage medium may also be computer-readable storage medium distributed amongst multiple computer systems or computing devices.

'Computer memory' or 'memory' is an example of a computer-readable storage medium. Computer memory is any memory which is directly accessible to a processor. Examples of computer memory include, but are not limited to: RAM memory, registers, and register files. References to 'computer memory' or 'memory' should be interpreted as possibly being multiple memories. The memory may for instance be multiple memories within the same computer system. the memory may also be multiple memories distributed amongst multiple computer systems or computing devices.

'Computer storage' or 'storage' is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa. References to 'computer storage' or 'storage' should be interpreted as possibly being multiple storage. The storage may for instance be multiple storage devices within the same computer system or computing device. The storage may also be multiple storages distributed amongst multiple computer systems or computing devices.

A 'computing device' or 'computer' as used herein encompasses to any device comprising a processor. A 'processor' as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. Many programs have their instructions performed by multiple processors that may be within the same computing device or which may even be distributed across multiple computing devices.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, one or more switches, one or more buttons, and accelerometer are all examples of user interface components which enable the receiving of information or data from an operator.

A 'hardware interface' as used herein encompasses a interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

A'database' as used herein encompasses a data file or repository which contains data that may be accessed by a processor. Examples of databases are, but are not limited to: a data file, a relational database, a file system folder containing data files, and a spreadsheet file.

In another aspect the invention provides for a method of modifying an obesity reduction aid according to an embodiment of the invention. The method comprises the step of recording the subject's weight periodically over a predetermined period of time. The method further comprises the step of determining a weight trend using the subject's weight recorded periodically. The method further comprises the step of recommending a chewing surface area reduction if the weight trend indicates a weight loss below a predetermined threshold.

In another embodiment the invention provides for a gobbling reduction training method. The method comprises the step of installing an obesity reduction aid according to an embodiment of the invention into the teeth of the subject. The method further comprises the step of feeding the subject. The method further comprises repeating this over a predetermined period of time. For instance the training may be formed over a period of one to three months.

It is understood that one or more of the claims and/or embodiments may be combined as long as the combined elements are not mutually exclusive.

### Brief description of the drawings

In the following embodiments of the invention are explained in greater detail, by way of example only, making reference to the drawings in which:
Fig. 1 shows a mandibular molar embedded in a gum of a subject;
Fig. 2 shows another view of the molar of Fig. 1 ;
Fig. 3 shows the mandibular molar of Fig. 1 covered by a lower plate;
Fig. 4 shows a maxillary molar opposite the mandibular molar of Fig. 3 where the molars are covered by an upper plate and a lower plate;
Fig. 5 shows a view of a lower plate showing the first chewing surface;
Fig. 6 shows an upper plate with a protruding ridge that forms the second chewing surface;
Fig. 7 shows a variant of the upper plate of Fig. 6 with an RFID chip installed;
Fig. 8 illustrates how the surface area of the protruding ridge in Fig. 6 may be reduced;
Fig. 9 illustrates an alternative as to how the surface area of the protruding ridge in Fig. 6 may be reduced;
Fig. 10 illustrates the upper plate of Fig 6 with the surface area indicated in Fig. 9 removed;
Fig. 11 shows a side view and a cross-sectional view of some teeth with a plate that uses clips for retention;
Fig. 12 shows a side view and a cross-sectional view of some teeth with a plate that uses alternative clips for retention;
Fig. 13 shows a method according to an embodiment of the invention; and
Fig. 14 shows an embodiment of an obesity reduction system.

### Detailed Description

Elements in these figures numbered in the same way are either equivalent elements or perform the same function. Elements which have been discussed previously will not necessarily be discussed in later figures if the function is equivalent.

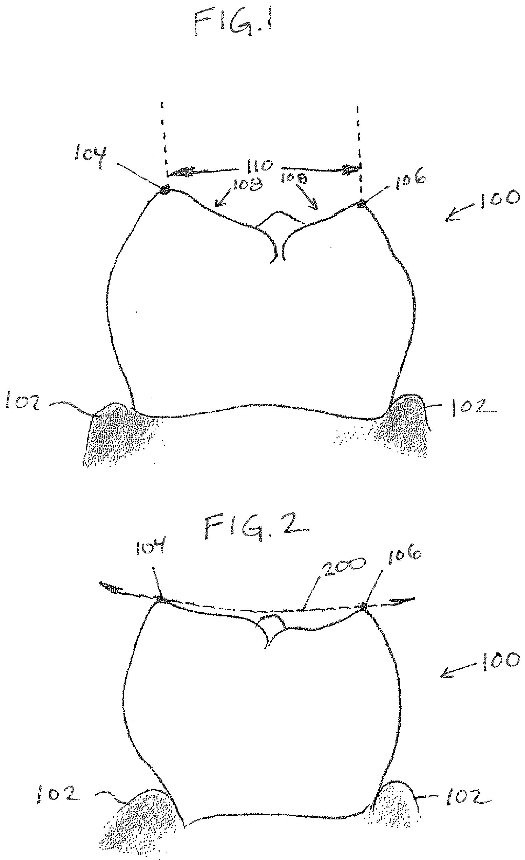

Fig. 1 shows a mandibular molar 100 embedded in a gum 102 of a subject. Point 104 is a high point of a first cusp. Point 106 is a high point of a second cusp. The space between the first point 104 and the second point 106 defines the occlusal surface. This is the surface which the subject uses to chew food. 110 is the distance between the first point 104 and the second point 106. This is the width of the occlusal surface above the tooth 100.

Fig. 2 shows the mandibular molar 100 again. In between the first point 104 and the second point 106 is a curved surface 200 which defines the occlusal plane. The occlusal plane 200 may provide an average or approximation of the occlusal surface 108 that is smooth.

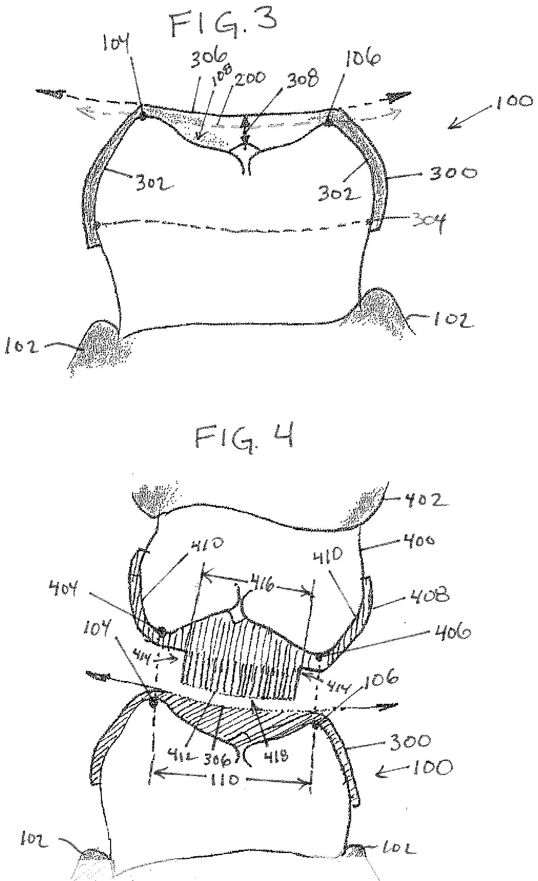

Fig. 3 shows the mandibular molar 100 covered by a lower plate 300. The lower plate 300 has been molded to fit the tooth 100. There is a first tooth mounting surface 302 that is formed to match the surface of the tooth 100. The lower plate 300 extends below the equator 304 of the tooth 100. This keeps the lower plate 300 from falling off the tooth 100. Above the tooth 100 there is the first chewing surface 306. It can be seen that the first chewing surface 306 is formed at an offset to the occlusal plane 200. The first chewing surface 306 does not cross through the occlusal plane 200. There is also an offset 308 of the chewing surface 306 from the occlusal surface 108.

Fig. 4 shows the mandibular molar 100 opposite a maxillary molar 400. The maxillary molar 400 is shown as being embedded in the gum 402 of the subject. As with the mandibular molar 100 the maxillary molar has several cusps. The high point of cusp 404 and the high point of the cusp 406 define two points 404, 406 which define the occlusal surface 108. There is an upper plate 408 positioned on the tooth 400. The upper plate 410 extends beyond the equator and has a form fitter on the tooth 400 also. The upper plate 400 is formed with a second tooth mounting surface 410 which fits the contours of the tooth 400 and holds the upper plate 408 in place. The upper plate 408 has a second chewing surface 412. The second chewing surface 412 is more built up than the lower plate 300. In other words, the second chewing surface 412 forms a stamp-like protrusion.

There is a step 414 on either side of this profile to create the protruding ridge 418. The protruding part 418 has a width 416. The width 416 is narrower than the width 110. As the width 416 is reduced there is less contact between the lower plate 300 and the upper plate 408. This would make it more difficult to chew. A dentist or other healthcare professional could use a grinding or cleaning tool to make the width 416 smaller on the entire upper plate 408 or just in certain regions. The first chewing surface 306 and the second chewing surface 412 both have a curvature which match each other. The curvature helps to position the first chewing surface and the second chewing surface 412 together. The curvature helps the teeth to naturally align during chewing. This makes eating with the obesity reduction aid easier for the subject.

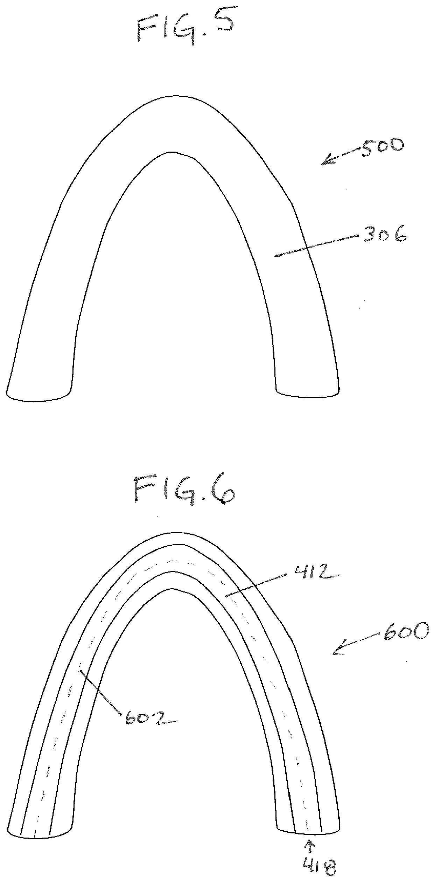

Fig. 5 shows a view of a lower plate 500 showing the first chewing surface 306. The lower plate 500 is smooth.

Fig. 6 shows an upper plate 600 with a protruding ridge 418 that forms the second chewing surface 412. The dashed line 602 shows a path along the maxillary teeth. The protruding ridge 418 follows the entire path 602. The combination of lower plate 500 and the upper plate 600 provides an example of an obesity reduction aid.

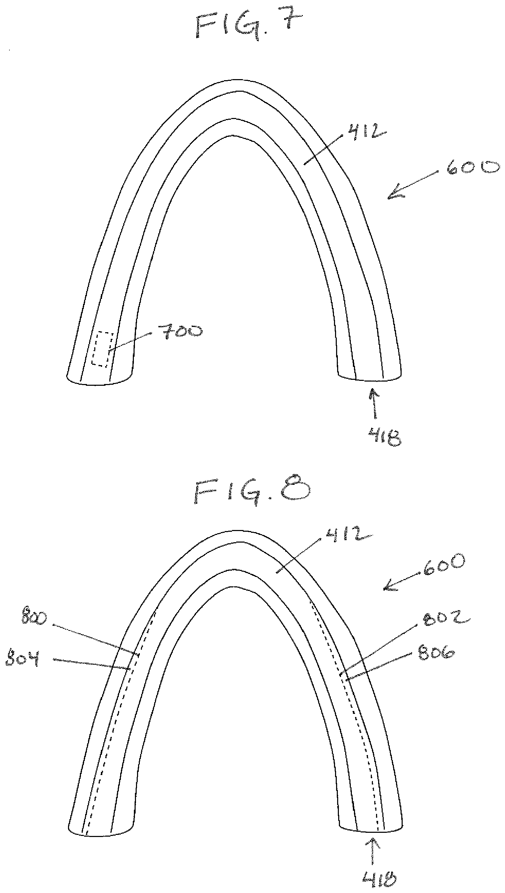

Fig. 7 shows a variant of the upper plate 600. In this embodiment there is an RFID chip 700 embedded in the upper plate 600. The RFID chip 700 may also be embedded in the lower plate as an alternative. Preferably, an RFID chip 700 is embedded both in the upper plate 600 and in the lower plate.

Fig. 8 shows the upper plate 600 again. Fig. 8 illustrates how the surface area of the second chewing surface 412 may be reduced. For instance if the subject is not losing weight rapidly enough or not losing weight at all the surface area of the second chewing surface 412 may be reduced. There is a first dashed line 800 and a second dashed line 802. These drawings show a position which may be ground to using a dental tool. Such a diagram as shown in Fig. 8 may for instance be displayed as part of the method of reducing the surface area of the chewing surface. The dashed line 800 indicates an area 804 which is suggested to be removed. The dashed line 802 suggests an area 806 to be removed from the second chewing surface 412. 804 and 806 may for instance be a surface area recommendation which may be displayed on a computer display or other display. In Fig. 8 the protruding ridge 418 is made narrower, thereby reducing the surface area.

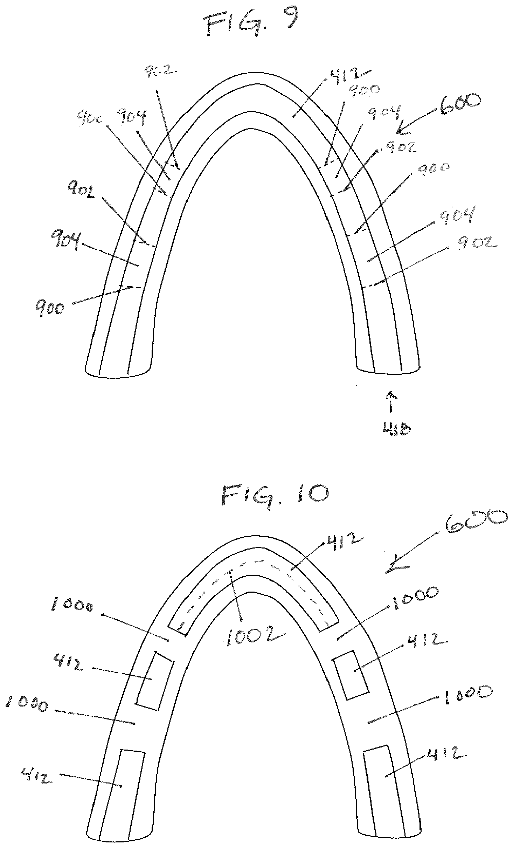

Fig. 9 shows an alternative view of the upper plate 600. Fig. 9 illustrates an alternative way in which the area of the second chewing surface 412 can be reduced. There are a series of lines 900 and 902 which indicate areas 904 to be removed.

Fig. 10 then shows the upper plate 600 after the areas 904 have been removed. The removed areas are labeled 1000. The second chewing surface 412 has been broken into several segments. The method of reducing the surface area of the second chewing surface 412 shown in Fig. 10 and in Fig. 8 may be combined. The dashed line 1002 is a path which follows the incisors of the maxillary teeth of the subject. Keeping the protruding ridge intact along a path 1002 prevents the wearer of the upper plate 600 from speaking with a lisp.

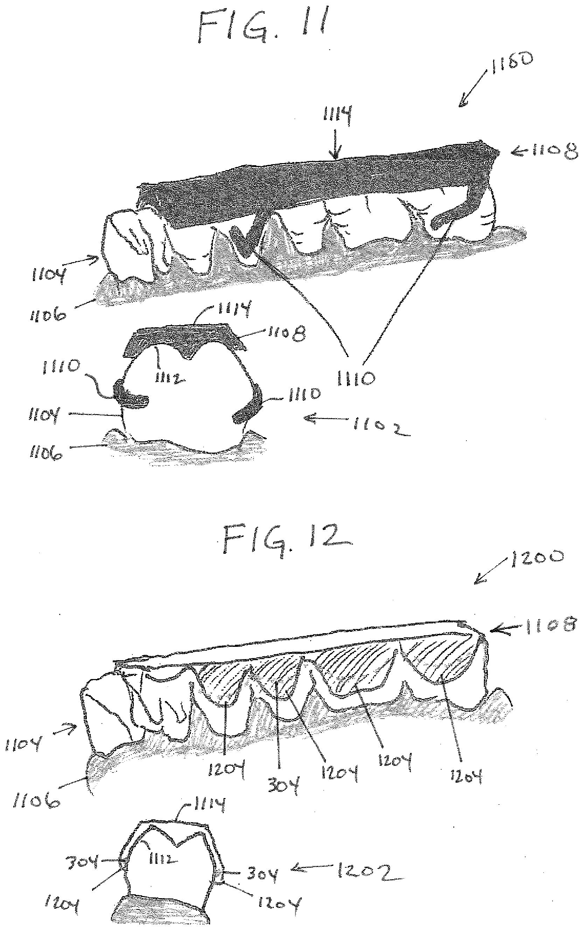

Fig. 11 shows a side view 1100 and a cross-sectional view 1102 of some teeth 1104 and gums 1106. The view in Fig. 11 is intended to be representative of both the upper plate and the lower plate. A portion of the plate 1108 which could be the upper plate or the lower plate is shown as resting on the teeth 1104. The portion of the plate 1108 has a tooth mounting surface 1112 which conforms to the shape of the teeth 1104. A chewing surface 1114 is visible. In this version the chewing surface 1114 is flat, as it is for the lower plate. However, the method of attaching the plate 1108 to the teeth 1104 is also relevant for the upper plate. There can be seen several clips 1110 which reach around and grab hold of individual teeth 1104 and hold the portion of the plate 1108 in position. The clip 1110 is formed out of the same elastic material as the portion of the plate 1108. The clips 1110 may be formed at the same time as the rest of the plate 1108 is formed. The portion of the plate 1108 and the clips 1110 are formed from one piece of material.

Fig. 12 shows a side view 1200 and a cross-sectional view 1202 of the teeth 1104 and gums 1106 again. In this example a similar portion of a plate 1108 is shown. In this example the clip 1204 is formed by extending a portion of the tooth mounting surface 1112 beyond the tooth equator 304. This portion 1204 functions as a clip 1204. The clip 1204 in this embodiment is also formed of one piece of the elastic material that is used to form the plate 1108.

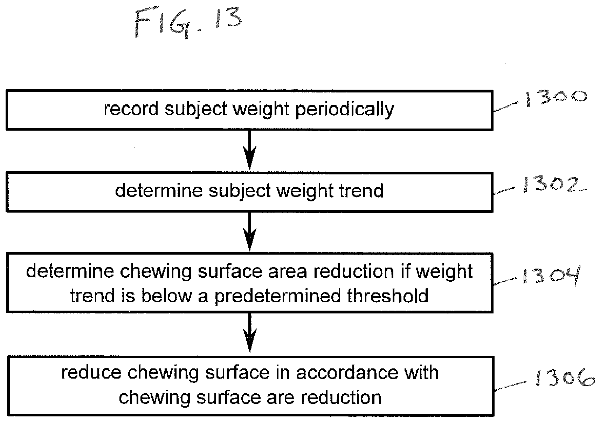

Fig. 13 shows the method according to an embodiment of the invention. First in step 1300 the subject weight is recorded periodically.

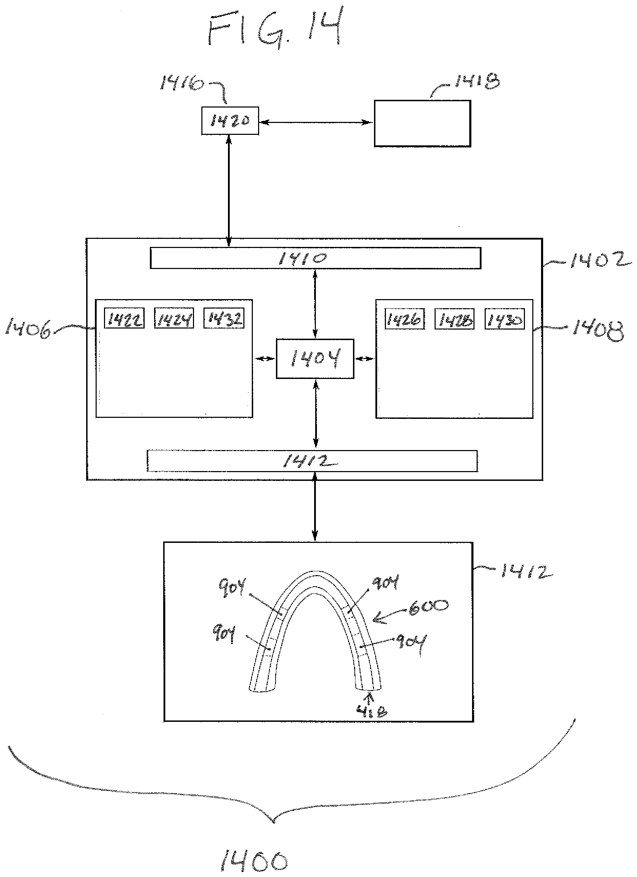

Fig. 14 shows an embodiment of an obesity reduction system 1400. The obesity reduction system comprises a computer 1402. The computer 1402 comprises a processor 1404. The processor 1404 is able to communicate with computer storage 1406, computer memory 1408, a hardware interface 1410 and a user interface 1412. The user interface 1412 comprises a display 1414. The hardware interface 1410 is shown as being connected to a memory 1416. The memory 1416 may be located in an RFID chip of an obesity reduction aid according to an embodiment of the invention or the memory 1410 may be located within the computer 1402 such as the computer storage 1406. The computer memory 1416 may also be located on an external device such as a mobile telephone device or other computer. There is a scale 1418 for measuring the weight of the subject periodically. The scale 1418 is shown as being in connection with the memory 1416 for adding the subject weight as a function of time to a weight database 1420. The weight database 1420 contains multiple weight measurements of the subject. In other embodiments the scale 1418 may be alternatively connected to the hardware interface 1410.

The computer storage 1404 is shown as containing a model of the first plate 1422 and a model of the second plate 1424. The two models 1422 and 1424 contain data descriptive of the first and second chewing surfaces. The computer memory 1408 is shown as containing a control module 1426. The control module 1426 contains computer-executable code which implements the obesity reduction system using the computer 1402. The computer memory 1408 is shown as further containing a weight trend module 1428. The weight trend module 1428 contains computer-executable code which enables the processor 1404 to determine a trend in the subject's weight using the weight database 1420.

The computer memory 1408 is shown as further containing a chewing area recommendation generation module 1430 which uses the weight trend generated by the weight trend module 1428 to generate a chewing surface area recommendation 1432 which is stored in the computer storage 1406. If the subject's weight is not decreasing beyond a certain rate then the chewing surface area recommendation may contain a recommendation to reduce the surface area of the second chewing surface. The chewing area recommendation generation module 1438 may be implemented in different ways. It may for example be implemented using rules programmed into it or it may be determined using empirical measurements and results from previous subjects.

The display 1412 shows a rendering of the chewing surface area recommendation 1432. The view in the display 1412 is identical with Fig. 9 . Areas labeled 904 indicate where a dentist or other healthcare provider may remove material from the protruding ridge 418 to reduce the surface area. Displaying this on the display 1412 may be beneficial for a dentist or technician because they can easily see how to modify the upper plate in order to optimize the subject's weight loss and training on how to properly chew.

### List of reference numerals

- 100: mandibular molar
- 102: gum
- 104: point of cusp
- 106: point of cusp
- 108: occlusal surface
- 110: width of occlusal surface
- 200: occlusal plane
- 300: lower plate
- 302: first tooth mounting surface
- 304: equator of tooth
- 306: first chewing surface
- 308: offset from occclusal surface
- 400: maxillary molar
- 402: gum
- 404: point of cusp
- 406: pont of cusp
- 408: upper plate
- 410: second tooth mounting surface
- 412: second chewing surface
- 414: step
- 416: width of chewing surface
- 418: protruding ridge
- 500: lower plate
- 600: upper plate
- 602: path along maxillary teeth
- 700: RFID chip
- 800: first dashed line
- 802: second dashed line
- 804: ridge to be removed
- 806: ridge to be removed
- 900: line
- 902: line
- 904: surface area to be removed
- 1000: removed area
- 1002: path
- 1100: side view
- 1102: cross sectional view
- 1104: teeth
- 1106: gum
- 1108: portion of plate
- 1110: clip
- 1112: tooth mounting surface
- 1114: chewing surface
- 1200: side view
- 1202: cross-sectional view
- 1204: clip
- 1400: obesity reduction system
- 1402: computer
- 1404: processor
- 1406: computer storage
- 1408: computer memory
- 1410: hardware interface
- 1412: user interface
- 1414: display
- 1416: memory
- 1418: scale
- 1420: weight database
- 1422: model of first plate
- 1424: model of second plate
- 1426: control module
- 1428: weight trend module
- 1430: chewing area recommendation generation module
- 1432: chewing surface area recommendation

## Claims

1. An obesity reduction aid comprising:
- a lower plate (300, 500) adapted for covering the mandibular teeth (100) of a subject, wherein the lower plate comprises a first elastic material, wherein the lower plate has a first chewing surface (306), wherein the first chewing surface is smooth, wherein the first chewing surface follows an average (200) of the occlusal surface (108) of the mandibular teeth of the subject;
- an upper plate (408, 600) adapted for covering the maxillary teeth (400) of the subject, wherein the upper plate comprises a second elastic material, wherein the upper plate has a second chewing surface (412), wherein the first chewing surface mates with the second chewing surface,
**characterised in that** the second chewing surface is formed by a protruding ridge (418), and
**in that** the surface area of the first chewing surface is larger than the surface area of the second chewing surface.

2. The obesity reduction aid of claim 1, wherein the protruding ridge is continuous along a path (602) following the maxillary teeth of the subject.

3. The obesity reduction aid of claim 1, wherein the protruding ridge is continuous along a path following the incisors (1002) of the maxillary teeth of the subject.

4. The obesity reduction aid of claim 1, 2, or 3, wherein the second chewing surface has an average width (110), wherein the protruding ridge has a width between 1/5 to 4/5 of the average width.

5. The obesity reduction aid of claim 1, 3, or 4, wherein the protruding ridge is at least partially discontinuous (1000) in the molar region of the subject.

6. The obesity reduction aid of any one of the preceding claims, wherein at least a portion of the second chewing surface has a convex profile, and wherein at least a portion of the first chewing surface has a concave profile.

7. The obesity reduction aid of claim 6, wherein the first chewing surface is above the occlusal plane, and wherein the first chewing surface spans (104, 106) the occlusal surface.

8. The obesity reduction aid of claim 6 or 7, wherein the first chewing surface has a first curvature, wherein the second chewing surface has a second curvature, and wherein the first curvature is equal to the second curvature.

9. The obesity reduction aid of claim 8, wherein the curvature is defined by the cusps of the mandibular molars of the subject.

10. The obesity reduction aid of claim 1, wherein the first chewing surface has a concave profile; wherein the lower plate has a first tooth mounting surface (302); wherein the first tooth mounting surface approximates the surface of the cusps of the mandibular teeth of the subject; wherein the concave profile is defined by the cusps of the teeth; wherein the lower plate is formed from a first single molded piece of the first elastic material; wherein the upper plate is formed from a second single molded piece of the second elastic material; wherein the lower plate comprises at least one first retention clip (1110, 1204) made from the first elastic material; wherein the upper plate comprises a second retention clip (1110, 1204) made from the second elastic material; wherein the first elastic material has an elastic modulus of between 2000 and 4000 MPa, preferably between 2600 and 3000 MPa; wherein the second elastic material has an elastic modulus of between 2000 and 4000 MPa, preferably between 2600 and 3000 MPa; and wherein the first chewing surface is defined by the curve of Spee.

11. The obesity reduction aid of any one of the preceding claims, wherein the lower plate comprises at least one first retention clip (1110, 1204) made from the first elastic material and/or wherein the upper plate comprises a second retention clip (1110, 1204) made from the second elastic material and/or wherein the lower plate is formed from a first single molded piece of the first elastic material and/or wherein the upper plate is formed from a second single molded piece of the second elastic material.

12. The obesity reduction aid of any one of the preceding claims, wherein the first elastic material has an elastic modulus of between 2000 and 4000 MPa and/or wherein the second elastic material has an elastic modulus of between 2000 and 4000 MPa.

13. The obesity reduction aid of any one of the preceding claims, wherein the obesity reduction aid comprises an RFID chip (700), wherein the RFID chip is operable for storing any one of the following: a patient identifier, a patient identity, a current weight, a starting weight, an ending weight, a weight history database, a three dimensional obesity reduction aid model, and combinations thereof.

14. An obesity reduction system comprising:
- an obesity reduction aid (500, 600) according to any one of the preceding claims;
- a computer memory (1408) for storing computer executable instructions and for storing a weight database (1420); and
- a processor (1404) for executing the instructions, wherein execution of the instructions causes the processor to:
- record (1300) a subject weight in the weight database;
- determine (1302, 1304) a chewing surface area reduction recommendation, using a model (1422) of the first chewing surface, a model (1424) of the second chewing surface, and the weight database;
- displaying (1306) the surface area reduction recommendation (804, 904, 1432) on a display (1412).

15. A method of modifying an obesity reduction aid according to any one of claims 1 through 13, wherein the method comprises the steps of:
- recording (1300) the subject weight periodically over a predetermined period of time;
- determining (1302) a weight trend using the subject weight recorded periodically; and
- recommending (1304) a chewing surface area reduction (804, 904, 1432) of the obesity reduction aid according to anyone of claims 1-13 if the weight trend indicates a weight loss below a predetermined threshold.

## Patentansprüche

1. Ein Hilfsmittel zur Reduktion von Adipositas mit:
- einer unteren Platte (300, 500), welche dazu eingerichtet ist, die Unterkieferzähne (100) einer Person zu bedecken, wobei die untere Platte ein erstes elastisches Material enthält, wobei die untere Platte eine erste Kaufläche (306) aufweist, wobei die erste Kaufläche glatt ist, wobei die erste Kaufläche einem Mittelwert der okklusalen Fläche (108) der Unterkieferzähne der Person folgt;
- einer oberen Platte (408, 600), welche dazu eingerichtet ist, die Oberkieferzähne (400) der Person zu bedecken, wobei die obere Platte ein zweites elastisches Material enthält, wobei die obere Platte eine zweite Kaufläche (412) aufweist, wobei die erste Kaufläche mit der zweiten Kaufläche zusammenpasst,
**dadurch gekennzeichnet, dass** die zweite Kaufläche durch einen hervorstehenden Grat (418) gebildet ist und
dadurch, dass der Oberflächenbereich der ersten Kaufläche größer als der Oberflächenbereich der zweiten Kaufläche ist.

2. Das Hilfsmittel zur Reduktion von Adipositas nach Anspruch 1, wobei der hervorstehende Grat durchgehend entlang eines Pfades (602) ist, welcher den Oberkieferzähnen der Person folgt.

3. Das Hilfsmittel zur Reduktion von Adipositas nach Anspruch 1, wobei der hervorstehende Grat durchgehend entlang eines Pfades ist, welcher den Schneidezähnen (1002) der Oberkieferzähne der Person folgt.

4. Das Hilfsmittel zur Reduktion von Adipositas nach Anspruch 1, 2 oder 3, wobei die zweite Kaufläche eine durchschnittliche Breite (110) aufweist, wobei der hervorstehende Grat eine Breite zwischen 1/5 und 4/5 der durchschnittlichen Breite aufweist.

5. Das Hilfsmittel zur Reduktion von Adipositas nach Anspruch 1, 3 oder 4, wobei der hervorstehende Grat im Bereich der Backenzähne der Person zumindest teilweise unterbrochen (1000) ist.

6. Das Hilfsmittel zur Reduktion von Adipositas nach irgendeinem der vorhergehenden Ansprüche, wobei wenigstens ein Abschnitt der zweiten Kaufläche ein konvexes Profil aufweist und wobei wenigstens ein Abschnitt der ersten Kaufläche ein konkaves Profil aufweist.

7. Das Hilfsmittel zur Reduktion von Adipositas nach Anspruch 6, wobei die erste Kaufläche oberhalb der okklusalen Fläche verläuft und wobei die erste Kaufläche die okklusale Fläche überspannt (104,106).

8. Das Hilfsmittel zur Reduktion von Adipositas nach Anspruch 6 oder 7, wobei die erste Kaufläche eine erste Krümmung aufweist, wobei die zweite Kaufläche eine zweite Krümmung aufweist und wobei die erste Krümmung gleich der zweiten Krümmung ist.

9. Das Hilfsmittel zur Reduktion von Adipositas nach Anspruch 8, wobei die Krümmung durch die Höcker der Unterkiefer-Backenzähne der Person definiert ist.

10. Das Hilfsmittel zur Reduktion von Adipositas nach Anspruch 1, wobei die erste Kaufläche ein konkaves Profil aufweist; wobei die untere Platte eine erste Zahnbefestigungsfläche (302) aufweist; wobei die erste Zahnbefestigungsfläche annähernd der Oberfläche der Höcker der Unterkieferzähne der Person entspricht; wobei das konkave Profil durch die Höcker der Zähne definiert ist; wobei die untere Platte durch ein erstes einzelnes gegossenes Teil aus dem ersten elastischen Material gebildet ist; wobei die obere Platte durch ein zweites einzelnes gegossenes Teil aus dem zweiten elastischen Material geformt ist; wobei die untere Platte wenigstens eine erste Rückhalteklammer (1110, 1204) aufweist, welche aus dem ersten elastischen Material hergestellt ist; wobei die obere Platte eine zweite Rückhalteklammer (1110, 1204) aufweist, welche aus dem zweiten elastischen Material hergestellt ist; wobei das erste elastische Material einen Elastizitätsmodul zwischen 2000 und 4000 MPa, vorzugsweise zwischen 2600 und 3000 MPa, aufweist; wobei das zweite elastische Material einen Elastizitätsmodul zwischen 2000 und 4000 MPa, vorzugsweise zwischen 2600 und-3000 MPa, aufweist; und wobei die erste Kaufläche durch die Spee-Kurve definiert ist.

11. Das Hilfsmittel zur Reduktion von Adipositas nach irgendeinem der vorhergehenden Ansprüche, wobei die untere Platte wenigstens eine erste Rückhalteklammer (1110, 1204) aufweist, welche aus dem ersten elastischen Material hergestellt ist, und/oder wobei die obere Platte eine zweite Rückhalteklammer (1110, 1204) aufweist, welche aus dem zweiten elastischen Material hergestellt ist, und/oder wobei die untere Platte durch ein erstes einzelnes gegossenes Teil aus dem ersten elastischen Material gebildet ist und/oder wobei die obere Platte durch ein zweites einzelnes gegossenes Teil aus dem zweiten elastischen Material gebildet ist.

12. Das Hilfsmittel zur Reduktion von Adipositas nach irgendeinem der vorhergehenden Ansprüche, wobei das erste elastische Material einen Elastizitätsmodul zwischen 2000 und 4000 MPa aufweist und/oder wobei das zweite elastische Material einen Elastizitätsmodul zwischen 2000 und 4000 MPa aufweist.

13. Das Hilfsmittel zur Reduktion von Adipositas nach irgendeinem der vorhergehenden Ansprüche, wobei das Hilfsmittel zur Reduktion von Adipositas einen RFID-Chip (700) aufweist, wobei der RFID-Chip betrieben werden kann, um irgendeines der folgenden zu speichern: einen Patientenbezeichner, eine Patientenidentität, ein momentanes Gewicht, ein Startgewicht, ein Endgewicht, eine Gewichtsverlaufsdatenbank, ein dreidimensionales Modell des Hilfsmittels zur Reduktion von Adipositas sowie Kombinationen davon.

14. Ein System zur Reduktion von Adipositas mit:
- einem Hilfsmittel zur Reduktion von Adipositas (500, 600) gemäß irgendeinem der vorhergehenden Ansprüche;
- einem Computerspeicher (1408) zum Speichern von auf einem Computer ausführbaren Anweisungen und zum Speichern einer Gewichtsdatenbank (1420); und
- einem Prozessor (1404) zum Ausführen der Anweisungen, wobei die Ausführung der Anweisungen den Prozessor veranlasst:
- ein Gewicht einer Person in die Gewichtsdatenbank aufzunehmen (1300);
- unter Verwendung eines Modells (1422) der ersten Kaufläche, eines Modells (1424) der zweiten Kaufläche und der Gewichtsdatenbank eine Empfehlung zur Reduktion des Kauflächenbereichs zu bestimmen (1302, 1304);
- die Empfehlung zur Reduktion des Kauflächenbereichs (804, 904, 1432) auf einer Anzeige (1412) anzuzeigen (1306).

15. Ein Verfahren zur Modifikation eines Hilfsmittels zur Reduktion von Adipositas nach irgendeinem der Ansprüche 1 bis 13, wobei das Verfahren die Schritte beinhaltet:
- periodisches Aufnehmen (1300) des Gewichts einer Person über eine vorbestimmte Zeitspanne;
- Bestimmen (1302) eines Gewichtstrends unter Verwendung des periodisch aufgenommenen Gewichts der Person; und
- Empfehlen (1304) einer Reduktion des Kauflächenbereichs (804, 904, 1432) des Hilfsmittels zur Reduktion von Adipositas gemäß irgendeinem der Ansprüche 1-13, wenn der Gewichtstrend einen Gewichtsverlust unter einer vorbestimmten Schwelle anzeigt.

## Revendications

1. Aide à la réduction de l'obésité qui comprend :
- une plaque inférieure (300, 500) adaptée pour recouvrir les dents mandibulaires (100) d'un sujet, la plaque inférieure comprenant un premier matériau élastique et possédant une première surface de mastication (306), la première surface de mastication étant lisse et suivant une moyenne (200) de la surface occlusale (108) des dents mandibulaires du sujet ;
- une plaque supérieure (408, 600) adaptée pour recouvrir les dents maxillaires (400) du sujet, la plaque supérieure comprenant un second matériau élastique et ayant une seconde surface de mastication (412), dans laquelle la première surface de mastication s'accouple avec la seconde surface de mastication, **caractérisée en ce que** la seconde surface de mastication est formée par une nervure saillante (418), et **en ce que** la superficie de la première surface de mastication est supérieure à la superficie de la seconde surface de mastication.

2. Aide à la réduction de l'obésité selon la revendication 1, dans laquelle la nervure en saillie est continue le long d'un trajet (602) qui suit les dents maxillaires du sujet.

3. Aide à la réduction de l'obésité selon la revendication 1, dans laquelle la nervure en saillie est continue le long d'un trajet qui suit les incisives (1002) des dents maxillaires du sujet.

4. Aide à la réduction de l'obésité selon la revendication 1, 2 ou 3, dans laquelle la seconde surface de mastication possède une largeur moyenne (110), dans laquelle la nervure en saillie possède une largeur comprise entre 1/5 et 4/5 de la largeur moyenne.

5. Aide à la réduction de l'obésité selon la revendication 1, 3 ou 4, dans laquelle la nervure en saillie est au moins partiellement discontinue (1000) dans la région des molaires du sujet.

6. Aide à la réduction de l'obésité selon l'une quelconque des revendications précédentes, dans laquelle au moins une partie de la seconde surface de mastication possède un profil convexe, et dans laquelle au moins une partie de la première surface de mastication possède un profil concave.

7. Aide à la réduction de l'obésité selon la revendication 6, dans laquelle la première surface de mastication se trouve au-dessus du plan occlusal, et dans laquelle la première surface de mastication s'étend (104, 106) sur la surface occlusale.

8. Aide à la réduction de l'obésité selon la revendication 6 ou 7, dans laquelle la première surface de mastication possède une première courbure, la seconde surface de mastication possède une seconde courbure, et la première courbure est égale à la seconde courbure.

9. Aide à la réduction de l'obésité selon la revendication 8, dans laquelle la courbure est définie par les pointes des molaires mandibulaires du sujet.

10. Aide à la réduction de l'obésité selon la revendication 1, dans laquelle la première surface de mastication possède un profil concave ; dans laquelle la plaque inférieure possède une première surface de montage de dents (302) ; dans laquelle la première surface de montage de dents s'approche de la surface des pointes des dents mandibulaires du sujet ; dans laquelle le profil concave est défini par les pointes des dents ; dans laquelle la plaque inférieure est formée d'une première pièce moulée en premier matériau élastique ; dans laquelle la plaque inférieure est formée d'une seconde pièce moulée en second matériau élastique ; dans laquelle la plaque supérieure comprend au moins un premier clip de maintien (1110, 1204) composé du premier matériau élastique ; dans laquelle la plaque inférieure comprend un second clip de maintien (1110, 1204) composé du second matériau élastique ; dans laquelle le premier matériau élastique possède un module d'élasticité compris entre 2000 et 4000 MPa, de préférence entre 2600 et 3000 MPa ; dans laquelle le second matériau élastique possède un module d'élasticité compris entre 2000 et 4000 MPa, de préférence entre 2600 et 3000 MPa ; et dans laquelle la première surface de mastication est définie par la courbe de Spee.

11. Aide à la réduction de l'obésité selon l'une quelconque des revendications précédentes, dans laquelle la plaque supérieure comprend au moins un premier clip de maintien (1110, 1204) composé du premier matériau élastique et/ou dans laquelle la plaque inférieure comprend un second clip de maintien (1110, 1204) composé du second matériau élastique et/ou dans laquelle la plaque inférieure est formée d'une première pièce moulée en premier matériau élastique et/ou dans laquelle la plaque inférieure est formée d'une seconde pièce moulée en second matériau élastique.

12. Aide à la réduction de l'obésité selon l'une quelconque des revendications précédentes, dans laquelle le premier matériau élastique possède un module d'élasticité compris entre 2000 et 4000 MPa et/ou dans laquelle le second matériau élastique possède un module d'élasticité compris entre 2000 et 4000 MPa.

13. Aide à la réduction de l'obésité selon l'une quelconque des revendications précédentes, qui comprend une puce RFID (700), dans laquelle la puce RFID est capable de stocker n'importe lequel de ce qui suit : un identifiant du patient, une identité du patient, un poids actuel, un poids de départ, un poids de fin, une base de données d'historique du poids, un modèle d'aide à la réduction de l'obésité en trois dimensions, et des combinaisons de ceux-ci.

14. Système de réduction de l'obésité qui comprend :
- une aide à la réduction de l'obésité (500, 600) selon l'une quelconque des revendications précédentes ;
- une mémoire informatique (1408) destinée à stocker des instructions exécutables par un ordinateur et à stocker une base de données de poids (1420) ; et
- un processeur (1404) destiné à exécuter les instructions, l'exécution des instructions permettant au processeur :
- d'enregistrer (1300) le poids d'un sujet dans la base de données de poids ;
- de déterminer (1302, 1304) une recommandation de diminution de la surface de mastication, à l'aide d'un modèle (1422) de la première surface de mastication, d'un modèle (1424) de la seconde surface de mastication, et de la base de données de poids ;
- d'afficher (1306) la recommandation de diminution de la surface (804, 904,1432) sur un écran (1412).

15. Procédé de modification d'une aide à la réduction de l'obésité selon l'une quelconque des revendications 1 à 13, qui comprend les étapes qui consistent à :
- enregistrer (1300) le poids du sujet de manière périodique sur une période de temps prédéterminée ;
- déterminer (1302) une tendance de poids en utilisant le poids du sujet enregistré de manière périodique ;
et
- recommander (1304) une diminution de la surface de mastication (804, 904, 1432) de l'aide à la réduction de l'obésité selon l'une quelconque des revendications 1 à 13 si la tendance de poids indique une perte de poids en-dessous d'un seuil prédéterminé.
